# EUROPEAN PATENT SPECIFICATION

(11) **EP 1 922 023 B1**
(45) Date of publication and mention of the grant of the patent: **14.10.2015**
(21) Application number: 06800894.5
(22) Date of filing: 07.08.2006
(51) Int. Cl.: A61F 2/00, A61F 2/02, A61F 13/00, A61B 17/08, A61B 17/00, A61B 17/12

(54) **IMPLANT FOR TREATING MYOCARDIAL RUPTURE**
IMPLANTAT ZUR BEHANDLUNG VON HERZMUSKELRISS
IMPLANT DESTINEE AU TRAITEMENT D'UNE RUPTURE MYOCARDIQUE

(30) Priority: 09.08.2005 US 199633
(43) Date of publication of application: 21.05.2008
(73) Proprietor: Cardiokinetix, Inc., Menlo Park, CA 94025 (US)
(72) Inventor: SHARKEY, Hugh R., Redwood City, CA 94062 (US); KHAIRKHAHAN, Alexander, Palo Alto, CA 94301 (US); NIKOLIC, Serjan D., San Francisco, CA 94121 (US); RADOVANCEVIC, Branislav, Houston, TX 77005 (US)
(74) Representative: Potter Clarkson LLP
(86) International application number: PCT/US2006/030745
(87) International publication number: WO 2007/021647

(56) References cited:
- WO-A2-2005/007031
- US-A1- 2005 015 109
- US-A1- 2005 154 252

## Description

### FIELD OF THE INVENTION

The present invention relates generally to the field of treating heart disease, particularly schemic coronary disease, and more specifically, to a device for partitioning a patient's heart chamber having a myocardial rupture or exhibiting characteristics of an incipient rupture.

### BACKGROUND OF THE INVENTION

An acute myocardial infarction (AMI) may lead to myocardial rupture. Myocardial rupture may also occur as a result of blunt and penetrating cardiac trauma, primary cardiac infection, primary and secondary cardiac tumors, infiltrative diseases of the heart, and aortic dissection. Mortality rates are extremely high unless early diagnosis and surgical intervention are provided rapidly. The consequences of myocardial rupture in the setting of AMI can be cardiac pericardial tamponade, ventricular septal defect (VSD), acute mitral regurgitation (MR), or formation of a pseudoaneurysm.

Cardiac tamponade is a clinical syndrome caused by the accumulation of fluid in the pericardial space, resulting in reduced ventricular filling and subsequent hemodynamic compromise. Cardiac tamponade is a medical emergency. The overall risk of death depends on the speed of diagnosis, the treatment provided, and the underlying cause of the tamponade.

The pericardium, which is the membrane surrounding the heart, is comprised of two layers. The parietal pericardium is the outer fibrous layer and the visceral pericardium is the inner serous layer with the pericardial spaced formed therebetween. The pericardial space normally contains 20-50 mL of fluid. Pericardial effusions can be serous, serosanguineous, hemorrhagic, or chylous.

The hemodynamic changes in the case of cardiac tamponade have been described to include three phases. Phase one involves the accumulation of pericardial fluid causing increased stiffness of the ventricle, requiring a higher filling pressure. In phase two fluid further accumulates resulting in reduced cardiac output. In phase three the pericardial and left ventricular (LV) filling pressures equilibrate, resulting in further decrease in cardiac output.

The development of tamponade may result in diminished diastolic filling as well as altered systemic venous return, both of which may result in reduced cardiac output.

Additionally, mechanical assist devices have been developed as intermediate procedures for treating patients in cardiogenic shock resulting from tamponade. Such devices include left ventricular assist devices and total artificial hearts. A left ventricular assist device includes a mechanical pump for increasing blood flow from the left ventricle into the aorta. Total artificial heart devices, such as the Jarvik heart, are usually used only as temporary measures while a patient awaits surgical repair of the lesion.

Surgical therapies for a hemodynamically unstable patient or one with recurrent tamponade include procedures such as pericardial centesis or surgical creation of a pericardial window involving open thoracotomy and/or pericardiotomy; creation of a pericardio-peritoneal shunt, and resection of the infarcted area and closure of the rupture zone with Teflon or Dacron patches or with the use of biological glues, are among the recommended surgical therapies. For a patient with a ventricular septal defect (VSD), surgical therapies include by directly closing or replacing of a patch, similar to treatment of tamponade. These procedures are highly invasive, risky and expensive and are commonly only done in conjunction with other procedures (such as heart valve replacement or coronary artery by-pass graft).

US 2005/0015109, on which the preamble of claim 1 is based, discloses a system for altering blood flow through the left ventricle that comprises a sealing element for sealing against an inner wall of the left ventricle. The sealing element is movable between collapsed and expanded configurations and comprises a body having a generally convex outer surface with an apex. The element has a cover with a number of support members secured to the cover. The support members extend generally towards the apex and are coupled to one another or to a central member at their distal ends at or near the apex. The proximal ends of the support members curve outwardly and are attached to a circumferential band.

In an alternative embodiment, the sealing element has support members of different lengths but no outwardly curved proximal ends.

### SUMMARY OF THE INVENTION

According to the present invention there is provided a device for treating a patient's heart according to claim 1.

Also disclosed, but not part of the claimed invention, is a method for the treatment of a patient's heart which has a myocardial rupture, and or stabilizing a traumatic rupture through the heart wall (e.g., stab wound). A myocardial infraction, (MI), may result in myocardial ruptures leading to pericardial tamponade, VSD, acute mitral regurgitation (MR), or formation of a pseudoaneurysm. The disclosed method is directed to treating a patient's heart having such a rupture (partial or complete); and/or an incipient rupture. The method includes separating the weakened or failed region to minimize the size and/or the effects of such a rupture or opening. The method at least partially restores of the hemodynamic competency of the chamber of the patient's heart which has or will soon have a rupture. The amount of pericardial fluid needed to impair the diastolic filling of the heart depends on the rate of fluid accumulation and the compliance of the pericardium. Rapid accumulation of fluid may have a much greater impact on increasing the pericardial pressure, thus severely impeding cardiac output, than fluid accumulation over a longer period. The device of the present invention, moreover, improves the diastolic function of the patient's heart.

In particular, the method includes partitioning a chamber (e.g., left or/or right ventricles) of the patient's heart into a main productive portion and a secondary non-productive portion which has a rupture or which exhibits the characteristics of an incipient rupture. This partitioning closes off the portion of the heart having the rupture or incipient rupture to prevent loss of blood from the chamber and to reduce the total volume of the heart chamber and thereby reducing the stress applied to weakened tissue of the patient's heart wall. As a result, the ejection fraction of the chamber is improved.

One disclosed method includes the use of a partitioning device having a partitioning membrane, preferably a reinforced partitioning device, with a pressure receiving surface, preferably concave, which defines in part the main productive portion of the partitioned heart chamber when disposed, preferably securely, within the patient's heart chamber.

The pressure receiving surface is formed from a flexible membrane that is reinforced by a radially expandable frame component formed of a plurality of ribs. The ribs of the expandable frame have proximal ends which are free, and distal ends which are preferably secured to a central hub to facilitate radial self expansion of the free proximal ends of the ribs away from a centerline axis. The distal ends of the ribs may be pivotally mounted to the hub and biased outwardly or fixed to the hub. The ribs are preferably formed from material such as superelastic NiTi alloy which allows for compressing the free proximal ends of the ribs toward the centerline axis and into a contracted configuration for delivery and self expansion when released for deployment upon release within the patient's heart chamber. The membrane may be of variable shape suitable to practice the present invention and aid in the treatment of the MI. In one embodiment the membrane has an eccentric shape to more particularly be configured for use in the upper portions of the ventricular septum.

The free proximal ends of the ribs are configured to engage, and preferably penetrate into, the tissue lining of the targeted heart chamber (i.e., heart chamber to be partitioned). The engagement, preferably penetration, of the proximal ends with the tissue lining of the heart chamber, enables the securing of a peripheral edge of the partitioning device to the heart wall and fixation of the partitioning device within the chamber so as to partition the chamber in a desired manner. Preferably, tissue penetrating proximal tips of the free proximal ends are configured to penetrate the tissue lining at an angle approximately perpendicular to the centerline axis of the partitioning device. The tissue penetrating proximal tips of the ribs may be provided with barbs, hooks and the like which prevent undesired withdrawal of the tips from the heart wall.

One or more strands extend the ribs at or close to an outer edge or periphery of the membrane to exert enough pressure to the flexible membrane periphery when the partitioning device is in an expanded configuration to provide an adequate seal between the membrane periphery and the lining of the heart wall. In one embodiment, a single strand or strands extend around essentially the entire periphery of the membrane so that the flexible periphery of the membrane between each pair of ribs is effectively sealed against the heart wall. The expansive strand or strands are formed from material which is stiffer than the flexible, unsupported material of the membrane to provide an outward expansive force or thrust to prevent formation of undesirable inwardly directed folds or wrinkles when the ribs of the partitioning device are in a contracted configuration. Suitable strand or strands are formed from materials such as polypropylene suture or supereleastic NiTi alloy wires. Such strands are typically about 0.13 to about 0.76 mm (about 0.005 to about 0.03 inch) in diameter to provide the requisite outward expansive force when placed in a circular position such as around the periphery of the membrane in less than completely expanded configuration.

The ribs in their expanded configuration angle outwardly from the hub and the free proximal ends curve outwardly so that the membrane is secured to the ribs of the expanded frame forming a trumpet-shaped, pressure receiving surface.

The partitioning membrane in the expanded configuration generally has radial dimension from about 10 to about 160 mm, preferably from about 50 to about 100 mm, as measured from the centerline axis. The membrane is preferably formed from flexible material or fabric such as expanded polytetrafluoroethylene (ePTFE).

In an embodiment, the partitioning device is designed to be oversized with respect to the chamber in which it is to be deployed so that the ribs of the device are under compression so that they can apply an outward force against the chamber wall. When the partitioning device is collapsed for delivery, the outwardly biased strand or strands ensure that there are no inwardly directed folds or wrinkles and that none are formed when the partitioning device is expanded for deployment within the heart chamber.

In one partitioning device design useful in the practice of the described methods, the free ends of the expansive strand or strands may be secured together and/or to the partitioning device. Alternatively, in another device design, the expansive strand or strands may be sufficiently long so that one or both free ends thereof extend out of the patient to facilitate collapse and retrieval of the partitioning device, if so desired. Pulling on the free ends of the strand extending out of the patient closes the expanded portion, i.e. the ribs and membrane, of the partitioning device to collapse the device as well as retrieving the collapsed partitioning device into an inner lumen of a guide catheter or other collecting device such as that described in co-pending application filed concurrently herewith entitled "Peripheral Seal for a Ventricular Partitioning Device", assigned to the assignee of the present invention.

The partitioning device preferably includes a supporting component or stem which has a length configured to extend distally to the heart wall surface to support the partitioning device within the heart chamber. In an embodiment, the supporting component has a plurality of pods or feet, preferably at least three, which distribute the force of the partitioning device about a region of the ventricular wall surface to minimize, preferably avoid, immediate or long term damage to the tissue of the heart wall, particularly compromised or necrotic tissue such as tissue of a myocardial infarct (MI) and the like. Pods of the support component extend radially and preferably are interconnected by struts or planes which help distribute the force over an expanded area of the ventricular surface.

The partitioning device may be delivered percutaneously or intraoperatively. One particularly suitable delivery catheter has an elongate shaft, a releasable securing device on a distal end of the shaft for holding the partitioning device on the shaft distal end and an expandable member such as an inflatable balloon on a distal portion of the shaft proximal to the shaft distal end to expand the interior surface of the collapsed partitioning device formed by the pressure receiving surface to effectuate the tissue penetrating tips or elements on the periphery of the partitioning device to sufficiently engage, preferably penetrate, the heart wall and to hold the partitioning device in a desired position to effectively partition the heart chamber. A suitable delivery device is described in co-pending application Serial No. 10/913,608, filed on August 5, 2004, assigned to the assignee of the present invention.

The described methods are easy to perform and provide for a substantially improved treatment of a diseased heart. As a result of the method, a more normal diastolic and systolic movement of a patient's diseased heart is achieved. Concomitantly, an increase in the ejection fraction of the patient's heart chamber is usually obtained. These and other advantages of the invention will become more apparent from the following detailed description of the invention and the accompanying exemplary drawings.

### BRIEF DESCRIPTION OF THE DRAWINGS

Figure 1A is a schematic view of a patient's heart having a myocardial infarct which may exhibit characteristics of an incipient rupture in the ventricular septum.
Figure 1B is a schematic view of the patient's heart of Figure 1A with a ventricular septal defect resulting from a rupture in the heart wall.
Figure 1C is a schematic view of the patient's heart of Figure 1B after treatment with a device according to the present invention, the schematic view not showing all features of the claimed device.
Figure 2A is a schematic view of a patient's heart exhibiting a myocardial infarct with free wall rupture of the left ventricular chamber.
Figure 2B is a schematic view of the patient's heart of Figure 2A with a left ventricular chamber tamponade.
Figure 2C is a schematic view of the patient's heart of Figure 2B after treatment with a device according to the present invention, the schematic view not showing all features of the claimed device.
Figure 3 is an elevational view of a partitioning device embodying features of the invention in an expanded configuration.
Figure 4 is a plan view of the partitioning device shown in Figure 3 illustrating the upper surface of the device.
Figure 5 is a bottom view of the partitioning device shown in Figure 3.
Figure 6 is a perspective view of the non-traumatic tip of the distally extending stem of the device shown in Figure 3.
Figure 7 is a partial cross-sectional view of a hub of the partitioning device shown in Figure 4 taken along the lines 7-7.
Figure 8 is a transverse cross-sectional view of the hub shown in Figure 7 taken along the lines 8-8.
Figure 9 is a longitudinal view, partially in section of a reinforcing rib and membrane at the periphery of the partitioning device shown in Figure 3.
Figure 10 is a schematic elevational view, partially in section, of a delivery system for the partitioning device shown in Figures 3 and 4.
Figure 11 is a transverse cross-sectional view of the delivery system shown in Figure 10 taken along the lines 11-11.
Figure 12 is an elevational view, partially in section, of the hub shown in Figure 7 secured to a helical coil of the delivery system shown in Figure 10.
Figures 13A-13E are schematic views of a patient's left ventricular chamber illustrating the deployment of the partitioning device shown in Figures 3 and 4 with the delivery system shown in Figure 10 to partition a patient's heart chamber (e.g., left ventricle) into a primary productive portion and a secondary, non-productive portion.
Figure 14 is a schematic view of the patient's heart after treatment utilizing a device embodying features of the present invention having an reinforced membrane with an eccentric peripheral base.

### DETAILED DESCRIPTION OF EMBODIMENTS OF THE INVENTION

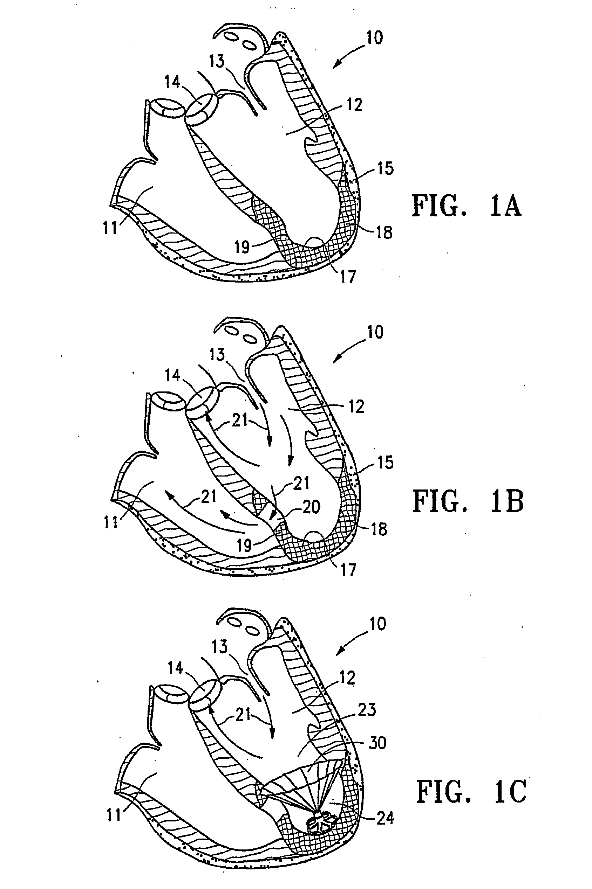

Figure 1A is a schematic illustration of a patient's heart 10 showing the right ventricle 11 and the left ventricle 12 with the mitral valve 13 and aortic valve 14. A pericardium membrane 15 is shown surrounding the heart 10. At least a portion of myocardium layer 17 of the left ventricle 12, as shown in Figure 1A, is exhibiting an area of infarct 18 ("MI") extending along a portion of ventricular septum wall 19 which separates the right and left ventricles and exhibits characteristics of an incipient rupture. Figure 1B illustrates the advancing of the infarct leading to the generation of a rupture or opening 20 in the septum wall 19, a condition referred to as VSD. As shown in Figure 1B oxygenated blood 21 flows directly to the right ventricle 11 through the septum opening 20. As a result of this movement, or shunting, at least two consequences are reached, firstly, the right portion of the heart works harder pumping a greater volume of blood than it normally would, and secondly, the amount of oxygenated blood in the left ventricle is reduced leading to a lower oxygen level to the other tissues of the body. Figure 1C illustrates the left ventricle 12 of Figure 1B after it has been partitioned, with the use of a partitioning device 30 according to the present invention and as described further below, into a main productive or operational portion 23 and a secondary, essentially non-productive portion 24. As can be seen from Figure 1C, with fluid path to the septum opening blocked or reduced, the normal flow of blood from the left ventricle to the rest of the body through the aortic valve is restored.

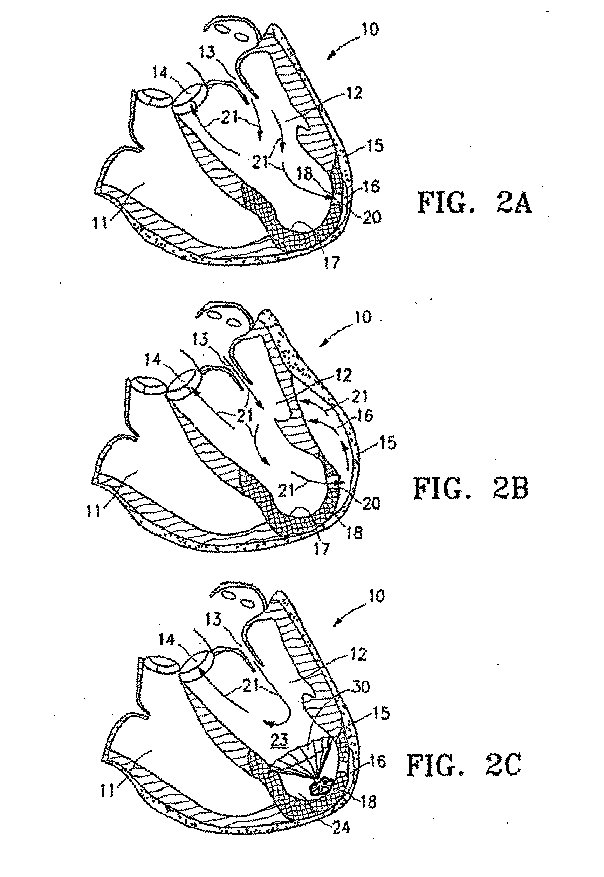

Figure 2A is a schematic illustration of the patient's heart 10 showing the right ventricle 11 and the left ventricle 12 with the mitral valve 13 and aortic valve 14.

The pericardium membrane 15 is shown surrounding the heart. A pericardium (pericardial complex) consists of an outer fibrous layer and an inner serous layer. The pericardial space 16 normally contains 20-50 mL of fluid. At least a portion of the myocardium layer 17 of the left ventricle 12, as shown in Figure 2A, is exhibiting the area of infarct 18 ("MI") extending along a portion of the left ventricle 12 resulting in the free wall rupture or opening 20 leading to a movement of blood 21 from the left ventricle into the pericardial space 16.

Figure 2B illustrates the advancing of the infarct leading to the rupture or opening 20 increasing in size. As shown in Figure 2B, the flow of the blood 21 into the pericardial space 16 increases over time leading to a greater accumulation of blood in the pericardial space. This movement and accumulation of blood in the pericardial space, a condition referred to as ventricular tamponade, results in reduced ventricular filling and subsequent hemodynamic compromise. Figure 2C illustrates the left ventricle 12 of Figure 2B after it has been partitioned, with the use of the partitioning device 30 according to the present invention, into the main productive or operational portion 23 and the secondary, essentially non-productive portion 24. As can be seen from Figure 1C, with fluid path to the pericardial space blocked or reduced, the normal flow of blood from the left ventricle to the rest of the body through the aortic valve is restored.

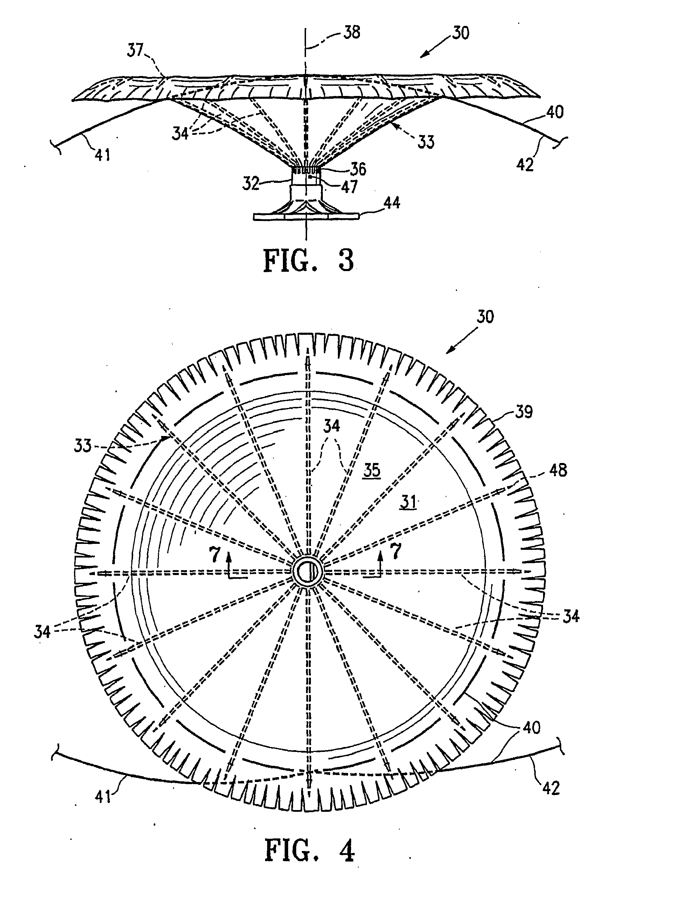

Figures 3-6 illustrate the partitioning device 30 which embodies features of the invention. The device 30 includes a partitioning membrane 31, a hub 32, preferably centrally located on the partitioning device, and a radially expandable reinforcing frame 33 formed of a plurality of ribs 34. Preferably, at least part of the partitioning membrane 31 is secured to a proximal or pressure receiving side 35 of the frame 33 as shown in Figure 3. The ribs 34 have distal ends 36 which are secured to the hub 32, and free proximal ends 37 which are configured to curve or flare away from a center line axis 38 at least upon expansion of the partitioning device. Radial expansion of the free proximal ends 37 unfurls the membrane 31 secured to the frame 33 so that the membrane presents the pressure receiving surface 35 which defines in part the productive portion 23 of the patient's partitioned heart chamber, as discussed with reference to Figures 1A-1C and 2A-2C. A peripheral edge 39 of the membrane 31 maybe serrated as shown.

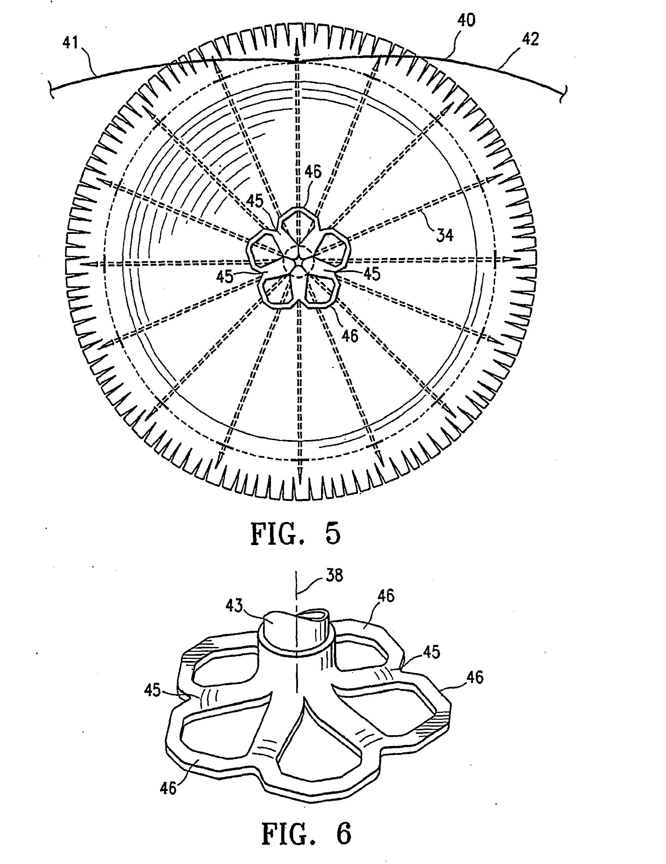

A continuous expansive strand 40 extends around the periphery of the membrane 31 on the pressure receiving side 35 thereof to apply pressure to the pressure side of the flexible material of the membrane to effectively seal the periphery of the membrane against the wall of the ventricular chamber. Ends 41 and 42 of the expansive strand 40 are shown extending away from the partitioning device in Figures 3 and 5. The ends 41 and 42 may be left unattached or may be secured together, e.g. by a suitable adhesive, to the membrane 31 itself. While not shown in detail, the membrane 31 has a proximal layer secured to the proximal faces of the ribs 34 and a distal layer secured to the distal faces of the ribs in a manner described in co-pending application Serial No. 10/913,608, filed on August 5, 2004, assigned to the assignee of the present invention.

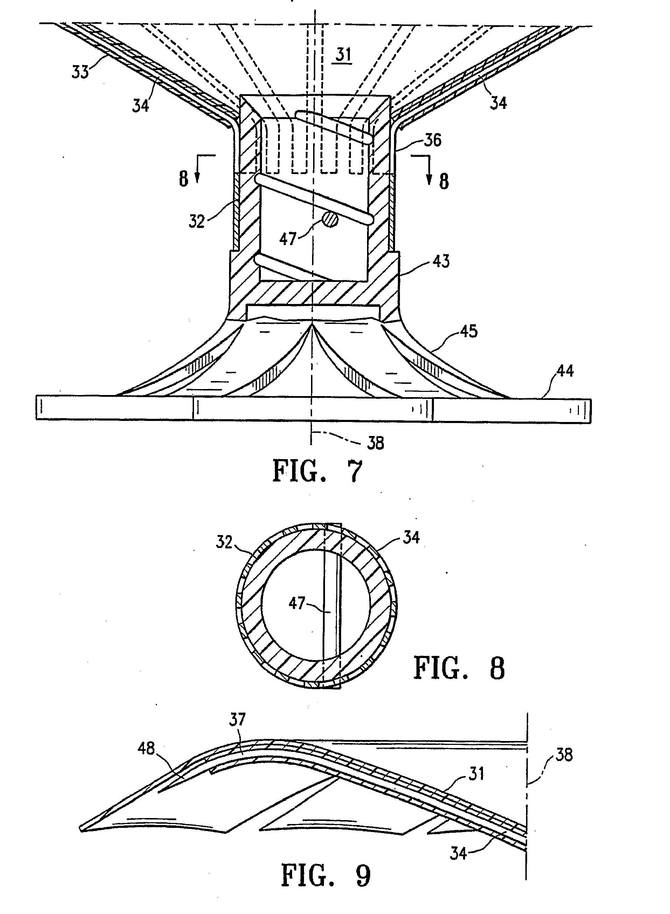

The hub 32 shown in Figures 6 and 7 preferably has a distally extending stem 43 with a non-traumatic support component 44. The support component 44 has a plurality of pods or feet 45 extending radially away from the center line axis 38 and the ends of the feet 45 are secured to struts 46 which extend between adjacent feet. A plane of material (not shown) may extend between adjacent feet 45 in a web-like fashion to provide further support in addition to or in lieu of the struts 46.

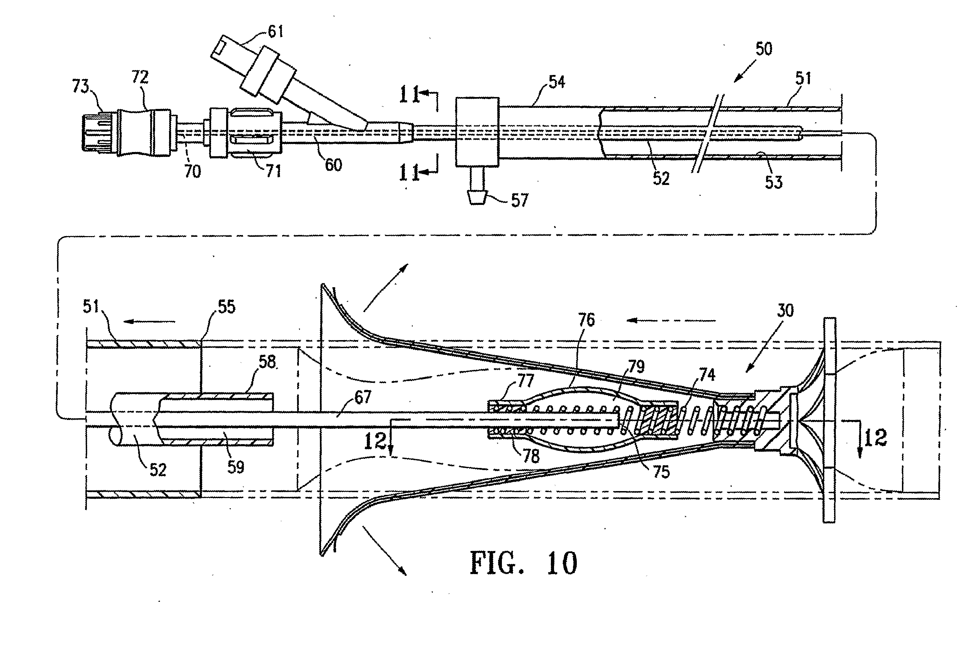

As shown in Figure 7, the distal ends 36 of the ribs 34 are secured within the hub 32 and, as shown in Figure 8, a transversely disposed connector bar 47 is secured within the hub which is configured to secure the hub 32 and thus the partitioning device 30 to a delivery system such as that shown in Figures 10-12.

Figure 9 illustrates the curved free proximal ends 37 of ribs 34 which are provided with sharp tip elements 48 configured to engage, and preferably penetrate into, the wall of the heart chamber and hold the partitioning device 30 in a deployed position within the patient's heart chamber so as to partition the ventricular chamber into a productive portion and a non-productive portion, as described above with reference to Figures 1A-1C and 2A-2C.

The connector bar 47 of the hub 32, as will be described later, allows the partitioning device 30 to be connected to the non-traumatic component 44 which can be secured to a delivery catheter for delivery and to be released from the delivery system within the patient's heart chamber. The distal ends 36 of the reinforcing ribs 34 are secured within the hub 32 in a suitable manner or they may be secured to the surface defining the inner lumen of the hub or they may be disposed within channels or bores in the wall of the hub 32. The distal end 36 of the ribs 34 are preshaped so that when the ribs are not constrained, other than by the membrane 31 secured thereto (as shown in Figures 3 and 4), the free proximal ends 37 thereof expand to a desired angular displacement, away from the centerline axis 38, of about 20° (degree) to about 90°, preferably about 50° to about 80°. The unconstrained diameter of the partitioning device 30 is preferably greater than the diameter of the heart chamber at the deployed location of the partitioning device so that an outward force is applied to the wall of the heart chamber by the at least partially expanded ribs 34 during systole and diastole so that the resilient frame 33 augments the heart wall movement.

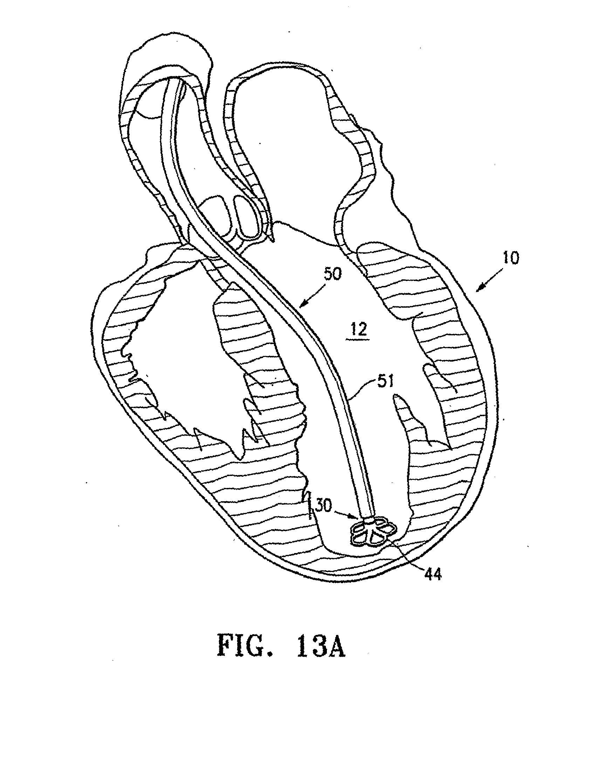

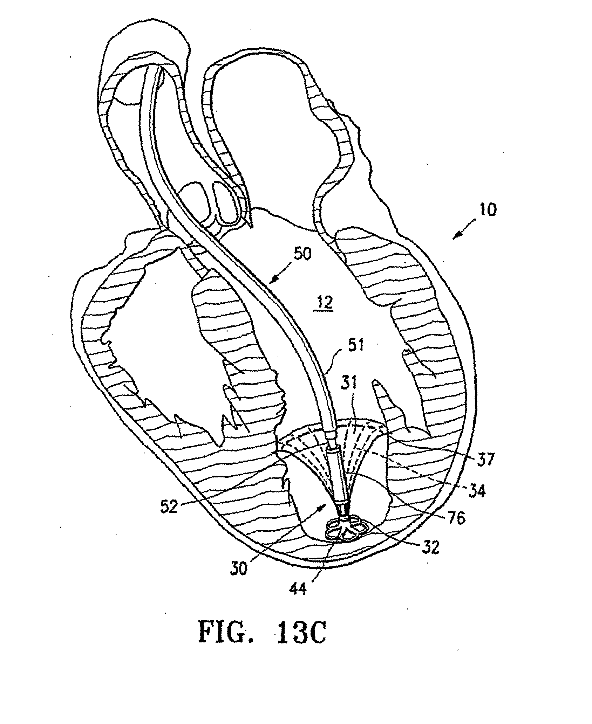

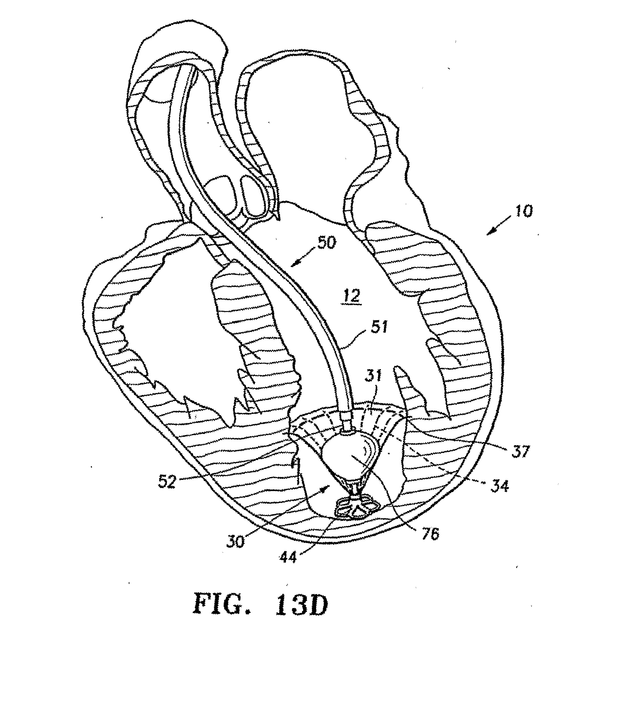

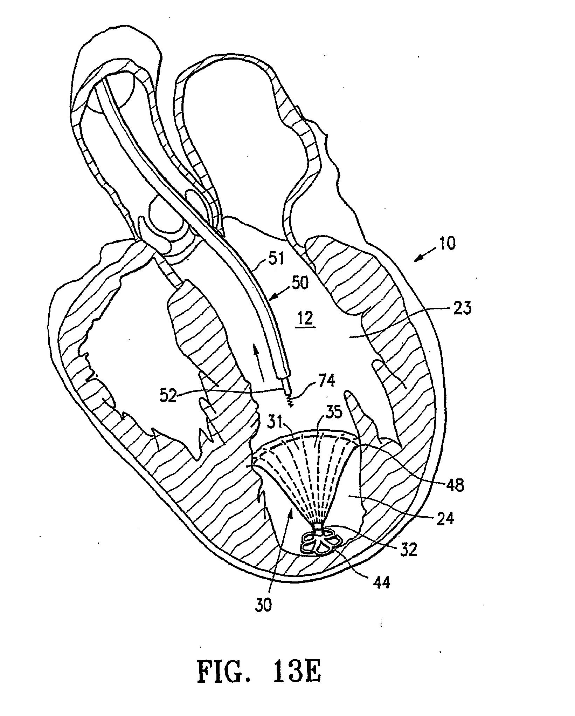

Figures 10-12 illustrate one suitable delivery system 50 for delivering the partitioning device 30, shown in Figures 3 and 4; into a patient's heart chamber and deploying the partitioning device to partition the heart chamber as shown in Figures 13A-13E. The delivery system 50 includes a guide catheter 51 and a delivery catheter 52. For purposes of clarity, as shown in Figures 13A-13E, the heart chamber is shown without rupture or openings 20 (as shown in Figures 1A-1C and Figures 2A-2C). The device may be used after the myocardial infarct has led to the creation of rupture or openings (such as 20) in the heart chamber, or in the case of an incipient rupture, prior to the creation of the rupture or openings as a means to minimize the size and/or the effects of rupture or opening.

The guide catheter 51 has an inner lumen 53 extending between proximal and distal ends, 54 and 55. A flush port 57 on the proximal end 54 of guide catheter 51 is in fluid communication with the inner lumen 53 for injecting therapeutic or diagnostic fluids thereto.

The delivery catheter 52 has an outer shaft 58 with an interior 59, and an adapter 60 at a proximal end thereof with a proximal injection port 61 which is fluid communication with interior 59 for injecting therapeutic or diagnostic fluids thereto. A hemostatic valve (not shown) may be provided at the proximal end 54 of the guide catheter 51 to seal about the outer shaft 58 of the delivery catheter 52.

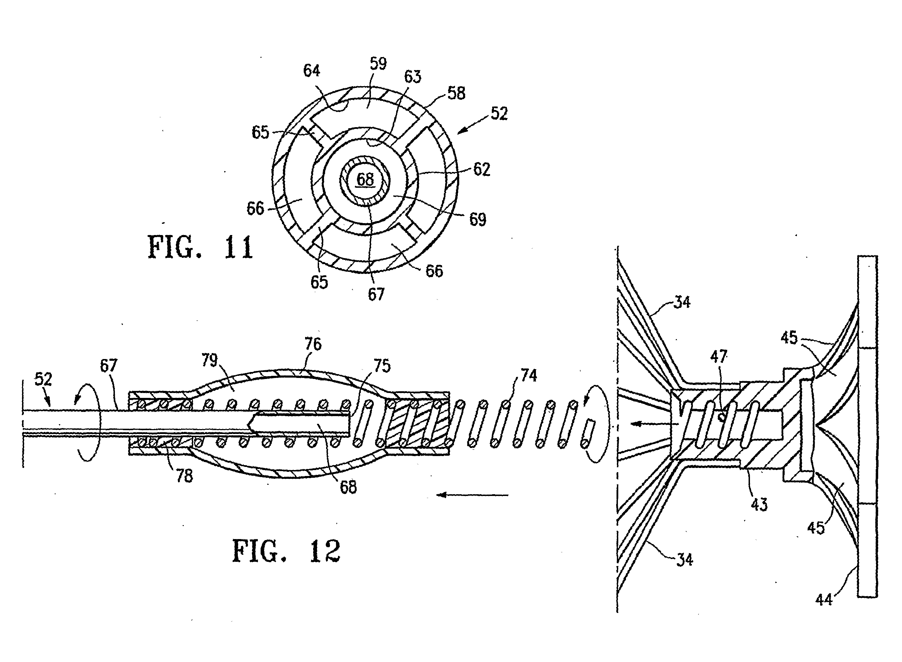

As shown in more detail in Figure 11, the outer shaft 58 has an inner shaft 62 with an interior 63, and is disposed within the interior 59 of the outer shaft and is secured to an inner surface 64 of the outer shaft 58 by webs 65 which extend along a substantial length of the inner shaft 62. The webs 65 define in part passageways 66 formed between the inner and outer shafts 62 and 58. The injection port 61 is in fluid communication with passageways 66 for directing therapeutic and/or diagnostic fluids thereto.

A torque shaft 67, preferably formed from hypotubing (e.g., stainless steel or superelastic NiTi) and having an inner lumen 68, is rotatably disposed within an inner lumen 69 of the inner shaft 62, and is secured at a proximal end 70 thereof within an adapter 71 with a rotating knob 72.

A balloon inflation port 73, preferably proximal to the rotating knob 72, is in fluid communication with the inner lumen 68 of the torque shaft 67.

A helical coil screw 74 is secured to a distal end 75 of the torque shaft 67 and rotation of the torque knob 72 on the proximal end 70 of the torque shaft 67 rotates the screw 74 on the distal end 75 of torque shaft 67 to facilitate deployment of the partitioning device 30. An inflatable balloon 76 at its proximal end 77 is sealingly secured (e.g., by way of adhesive 78) about the torque shaft 67 proximal to the distal end 75 of the torque shaft and has an interior 79 in fluid communication with the inner lumen 68 of the torque shaft 67. Inflation fluid may be delivered to the interior 79 of the balloon through port 73. Inflation of the balloon 76 by inflation fluid through port 73 facilitates securing the partitioning device 30 to the heart wall.

As shown in Figures 13A through 13E, the partitioning device 30 is delivered through the delivery system 50 which includes the guide catheter 51 and the delivery catheter 52. The partitioning device 30 is collapsed to a first delivery configuration which has small enough transverse dimensions to be slidably advanced through the inner lumen 53 of the guide catheter 51. Preferably, the guide catheter 51 has been previously percutaneously introduced and advanced through the patient's vasculature, such as the femoral artery, in a conventional manner to the desired heart chamber, such as the left ventricle 12. The delivery catheter 52 with the partitioning device 30 attached is advanced through the inner lumen 53 of the guide catheter 51 until the partitioning device 30 is ready for deployment from the distal end of the guide catheter 51 into the patient's heart chamber, such as left ventricle 12, to be partitioned.

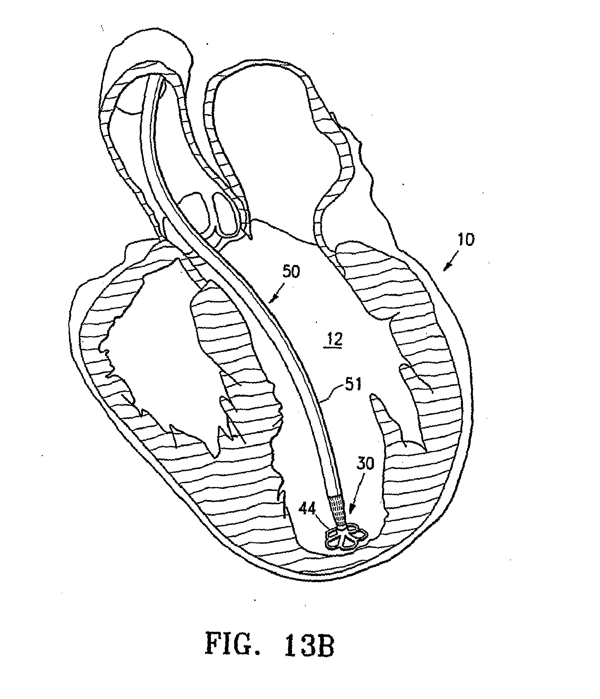

The partitioning device 30 mounted on the screw 74 is urged partially out of the inner lumen 53 of the guide catheter 51 until the support component 44 of the hub 32 engages the heart wall as shown in Figure 13B with the free proximal ends 37 of the ribs 34 in a contracted configuration within the guide catheter. The guiding catheter 51 is withdrawn while the delivery catheter 52 is held in place until the proximal ends 37 of the ribs 34 exit a distal end 55 of the guiding catheter 51. The free proximal ends 37 of ribs 34 expand outwardly to press the sharp proximal tips 48 of the ribs 34 against and preferably into the tissue lining the heart chamber.

With the partitioning device deployed within the heart chamber and preferably partially secured therein, inflation fluid is introduced through the inflation port 73 into the inner lumen 68 of the torque shaft 67 and into the balloon interior 79 to inflate the balloon 76. The inflated balloon 76 presses against the pressure receiving surface 35 of the membrane 31 of the partitioning device 30 to ensure that the sharp proximal tips 48 are pressed well into the tissue lining the heart chamber.

With the partitioning device 30 properly positioned within the heart chamber, the knob 72 on the torque shaft 67 is rotated (e.g., counter-clockwise) to disengage the helical coil screw 74 of the delivery catheter 52 from the stem 43 of the non-traumatic support component. The counter-clockwise rotation of the torque shaft 67 rotates the helical coil screw 74 which rides in the stem 43 of non-traumatic support component secured within the hub 32. Once the helical coil screw 74 disengages, the stem 43, the delivery system 50, including the guide catheter 51 and the delivery catheter 52, may then be removed from the patient.

The partitioning device 30 partitions the patient's heart chamber, such as left ventricle 12, into the main productive or operational portion 23 and the secondary, essentially non-productive portion 24. The operational portion 23 is much smaller than the original ventricular chamber and provides for an improved ejection fraction. The partitioning increases the ejection fraction and provides an improvement in blood flow. Over time, the non-productive portion 24 may fill first with thrombus and subsequently with cellular growth. Bio-resorbable fillers such as polylactic acid, polyglycolic acid, polycaprolactone and copolymers and blends thereof may be employed to initially fill the non-productive portion 24. Fillers may be suitably supplied in a suitable solvent such as dimethylsulfoxide (DMSO). Other materials which accelerate tissue growth or thrombus may be deployed in the non-productive portion 24 as well as non-reactive fillers. It should be noted that although the present figures describe the treatment of the left ventricle, the same can be applied to other chambers of the heart.

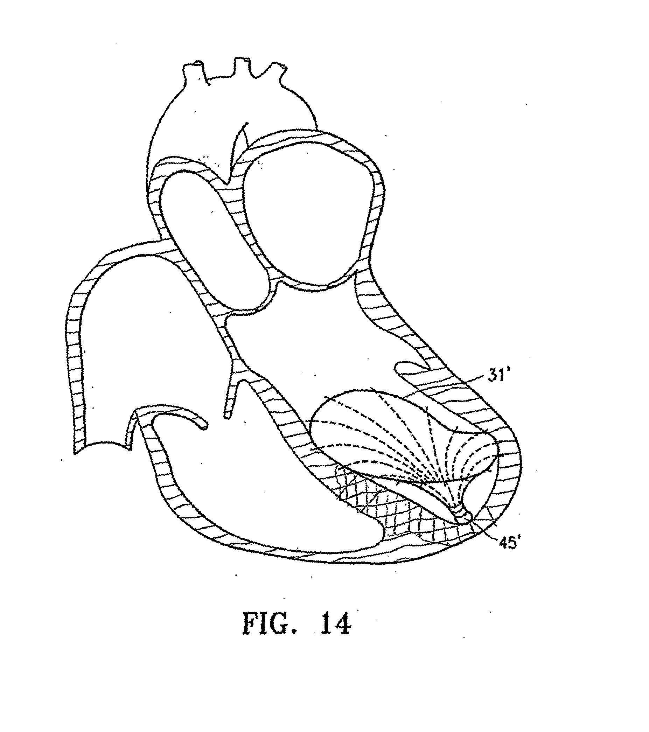

Figure 14 illustrates an alternative design in which the partitioning device 30' is provided with an eccentric-shaped membrane 31' which is well suited for treating VSD lesions that may occur further up (more proximal) the ventricular septum because of the different anatomical features and physiologic action of the ventricular septum versus the anterior free wall. The septal wall primarily moves in and out only, relative to the chamber, versus the free wall that has a rotation component to its excursion. Secondly, the outflow track which comprises the upper half of the ventricular septal wall below the aortic valve has very little or no trebeculation. It is particularly well suited for placement of the device placed to address necrotic failure of the tissue of the ventricular septum. In the embodiment shown in Figure 14, the device is shown with a nubbin foot 45' (and not the extended stem foot) allowing the device to sit more distally and intimately with the apex.

The details of the partitioning device 30' are essentially the same as in the previous embodiments and elements in this alternative embodiment are given the same reference numbers but primed as similar elements in the previously discussed embodiments. The partitioning device 30' forms a conical shape as in the previously discussed embodiments but the peripheral base of the conical shape which engages the wall that has a first dimension in a first direction greater than a second dimension in a second direction. Preferably, the second direction is at a right angle with respect to the first direction. The lengths of the ribs 34' are adjusted to provide the desired shape to the periphery of the device which engages the interior of the heart chamber.

The partitioning device 30 (and 31') may be conveniently formed by the method described in co-pending application Serial No. 10/913,608 assigned to the assignee of the present invention.

While porous ePTFE material is preferred, the membrane 31 may be formed of suitable biocompatible polymeric material which includes Nylon, PET (polyethylene terephthalate) and polyesters such as Hytrel. The membrane 31 is preferably foraminous in nature to facilitate tissue ingrowth after deployment within the patient's heart. The delivery catheter 52 and the guiding catheter 51 may be formed of suitable high strength polymeric material such as PEEK (polyetheretherketone), polycarbonate, PET, Nylon, and the like. Braided composite shafts may also be employed.

To the extent not otherwise described herein, the various components of the partitioning device and delivery system may be formed of conventional materials and in a conventional manner as will be appreciated by those skilled in the art.

While particular forms of the invention have been illustrated and described herein, it will be apparent that various modifications and improvements can be made to the invention. Moreover, individual features of embodiments of the invention may be shown in some drawings and not in others, but those skilled in the art will recognize that individual features of one embodiment of the invention can be combined with any or all the features of another embodiment. Accordingly, it is not intended that the invention be limited to the specific embodiments illustrated.

## Claims

1. A device (30) for treating a patient's heart comprising:
a plurality of radially expandable ribs (34) coupled at their distal ends (36) and outwardly curved at their free proximal ends (37); and
a membrane (31) coupled to said ribs;
said device being configured to be endovascularly delivered to a chamber of a heart,
**characterized in that**:
the device comprises an outwardly biased strand (40) threaded through the membrane, wherein the outwardly biased strand extends between and around the ribs and around the periphery (39) of the membrane to apply outward pressure to the membrane, and
the radially expandable ribs are of different lengths.

2. The device of claim 1, wherein the strand (40) is offset from an outer edge of the membrane (31).

3. The device of claim 1, wherein a proximal periphery of said ribs (34) is noncircular.

4. The device of claim 1 further comprising one or more anchor elements (48) at each of said proximal ends (37) of said different length ribs (34) adapted to secure said device (30) to a selected area of a wall within said heart.

5. The device of claim 1, wherein said device (30) once deployed adjustably fits into an irregularly shaped area of said heart.

6. The device of claim 1, wherein said device (30) once deployed is adapted for reducing a volume of said chamber.

7. The device of claim 1, wherein said device (30) once deployed is adapted for partitioning said chamber of said heart into a functional portion and a non-functional portion.

8. The device of claim 1, wherein said different length ribs (34) are self expandable upon deployment.

9. The device of claim 1, wherein said radially expandable ribs (34) expand to a proximal diameter of about 5mm to about 80 mm.

10. The device of claim 1, wherein said different length ribs (34) of said device (30) provide an outward force against a wall of said chamber.

11. The device of claim 1, wherein said device (30) once deployed augments movement of a wall of said chamber.

12. The device of claim 1, wherein said device (30) is adapted for reducing acute mitral regurgitation.

13. The device of claim 1 further comprising a stem (43) extending distally from said distal ends (36) of said different length ribs (34) wherein said stem is adapted to space said device (30) a selected distance from a wall of said chamber of said heart.

14. The device of claim 2, wherein said membrane (31) forms a pressure receiving surface (35).

15. The device of claim 4, wherein the or each anchor element (48) pierces tissue at an angle of not more than 45° from a center line axis (38) of said device (30).

16. The device of claim 7, wherein said non-functional portion comprises a myocardial rupture.

17. The device of claim 7, wherein said device (30) is adapted for partitioning a myocardial rupture that is not located at an apex of a ventricle.

18. The device of claim 7, wherein said non-functional portion comprises a pseudoaneurysm.

19. The device of claim 9, wherein said proximal diameter of said device (30) is oversized compared to a diameter of said chamber of said heart.

20. The device of claim 13, wherein said stem (43) comprises a non-traumatic support component (44) connected at its distal end.

21. The device of claim 14, wherein said pressure receiving surface (35) is concave at its distal end.

22. The device of claim 16, wherein said myocardial rupture is a ventricular septal defect.

23. The device of claim 16, wherein said myocardial rupture is cardiac pericardial tamponade.

24. The device of claim 20, wherein said non-traumatic support component (44) comprises a plurality of feet (45) adapted to distribute force of said device (30) about a region of said wall of said chamber.

## Patentansprüche

1. Vorrichtung (30) zum Behandeln eines Herzens eines Patienten, Folgendes umfassend:
mehrere radial erweiterbare Rippen (34), an ihren distalen Enden (36) gekoppelt und an ihren freien proximalen Enden (37) nach außen gekrümmt; und
eine Membran (31), gekoppelt mit den Rippen;
wobei die Vorrichtung konfiguriert ist, endovaskulär in eine Kammer eines Herzens eingebracht zu werden,
**dadurch gekennzeichnet, dass**:
die Vorrichtung einen nach außen vorgespannten Draht (40) aufweist, der durch die Membran hindurch gefädelt ist, wobei der nach außen vorgespannte Draht sich zwischen den und um die Rippen sowie um den Umfang (39) der Membran erstreckt, um Druck nach außen auf die Membran aufzubringen, und
die radial erweiterbaren Rippen verschiedene Längen aufweisen.

2. Vorrichtung nach Anspruch 1, wobei der Draht (40) von einem äußeren Rand der Membran (31) aus versetzt ist.

3. Vorrichtung nach Anspruch 1, wobei ein proximaler Umfang der Rippen (34) nicht rund ist.

4. Vorrichtung nach Anspruch 1, ferner umfassend ein oder mehrere Ankerelemente (48) an jedem der proximalen Enden (37) der verschieden langen Rippen (34), angepasst zum Sichern der Vorrichtung (30) an einem ausgewählten Bereich einer Wand in dem Herzen.

5. Vorrichtung nach Anspruch 1, wobei sich die Vorrichtung (30) nach dem Einsetzen anpassbar in einen unregelmäßig geformten Bereich des Herzens einfügt.

6. Vorrichtung nach Anspruch 1, wobei die Vorrichtung (30) nach dem Einsetzen angepasst ist, ein Volumen der Kammer zu verringern.

7. Vorrichtung nach Anspruch 1, wobei die Vorrichtung (30) nach dem Einsetzen angepasst ist, die Kammer des Herzens in einen Funktionsabschnitt und einen funktionslosen Abschnitt einzuteilen.

8. Vorrichtung nach Anspruch 1, wobei die verschieden langen Rippen (34) nach dem Einsetzen selbsterweiternd sind.

9. Vorrichtung nach Anspruch 1, wobei die radial erweiterbaren Rippen (34) sich auf einen proximalen Durchmesser von etwa 5 mm bis etwa 80 mm erweitern.

10. Vorrichtung nach Anspruch 1, wobei die verschieden langen Rippen (34) der Vorrichtung (30) eine nach außen gegen eine Wand der Kammer wirkende Kraft bereitstellen.

11. Vorrichtung nach Anspruch 1, wobei die Vorrichtung (30) nach dem Einsetzen die Bewegung einer Wand der Kammer verstärkt.

12. Vorrichtung nach Anspruch 1, wobei die Vorrichtung (30) angepasst ist, akute Mitralklappeninsuffizienz zu verringern.

13. Vorrichtung nach Anspruch 1, ferner umfassend einen Stiel (43), der sich distal von den distalen Enden (36) der verschieden langen Rippen (34) erstreckt, wobei der Stiel angepasst ist, die Vorrichtung (30) um einen ausgewählten Abstand von einer Wand der Kammer des Herzens zu beabstanden.

14. Vorrichtung nach Anspruch 2, wobei die Membran (31) eine Druck aufnehmende Oberfläche (35) ausbildet.

15. Vorrichtung nach Anspruch 4, wobei das oder jedes Ankerelement (48) Gewebe in einem Winkel von höchstens 45° von einer Mittellinienachse (38) der Vorrichtung (30) durchstößt.

16. Vorrichtung nach Anspruch 7, wobei der funktionslose Abschnitt eine Myokardruptur umfasst.

17. Vorrichtung nach Anspruch 7, wobei die Vorrichtung (30) angepasst ist, eine Myokardruptur abzuteilen, die sich nicht an einem Apex einer Herzkammer befindet.

18. Vorrichtung nach Anspruch 7, wobei der funktionslose Abschnitt ein Pseudoaneurysma umfasst.

19. Vorrichtung nach Anspruch 9, wobei der proximale Durchmesser der Vorrichtung (30) verglichen mit einem Durchmesser der Kammer des Herzens vergrößert ausgebildet ist.

20. Vorrichtung nach Anspruch 13, wobei der Stiel (43) eine nicht traumatische Stützkomponente (44) umfasst, die an seinem distalen Ende angebaut ist.

21. Vorrichtung nach Anspruch 14, wobei die Druck aufnehmende Oberfläche (35) an ihrem distalen Ende konkav ist.

22. Vorrichtung nach Anspruch 16, wobei die Myokardruptur ein Ventrikelseptumdefekt ist.

23. Vorrichtung nach Anspruch 16, wobei die Myokardruptur eine Herzbeuteltamponade ist.

24. Vorrichtung nach Anspruch 20, wobei die nicht traumatische Stützkomponente (44) mehrere Füße (45) umfasst, die angepasst sind, die Kraft der Vorrichtung (30) über eine Region der Wand der Kammer zu verteilen.

## Revendications

1. Dispositif (30) destiné à traiter le coeur d'un patient, comprenant :
une pluralité de nervures pouvant se dilater radialement (34) couplés au niveau de leurs extrémités distales (36) et courbées vers l'extérieur au niveau de leurs extrémités proximales libres (37) ; et
une membrane (31) couplée auxdites nervures ;
ledit dispositif étant configuré pour être délivré de façon endovasculaire à une chambre d'un coeur,
**caractérisé en ce que** :
le dispositif comprend un brin sollicité vers l'extérieur (40) fileté à travers la membrane, dans lequel le brin sollicité vers l'extérieur s'étend entre et autour des nervures et autour de la périphérie (39) de la membrane afin d'appliquer une pression extérieure à la membrane, et
les nervures pouvant se dilater radialement sont de longueurs différentes.

2. Dispositif selon la revendication 1, dans lequel le brin (40) est décalé par rapport à un bord externe de la membrane (31).

3. Dispositif selon la revendication 1, dans lequel une périphérie proximale desdites nervures (34) est non circulaire.

4. Dispositif selon la revendication 1, comprenant en outre un ou plusieurs éléments d'ancrage (48) au niveau de chacune desdites extrémités proximales (37) desdites nervures (34) de longueur différente adaptés pour arrimer ledit dispositif (30) à une zone sélectionnée d'une paroi au sein dudit coeur.

5. Dispositif selon la revendication 1, dans lequel ledit dispositif (30), une fois déployé, s'ajuste de façon réglable dans une zone de forme irrégulière dudit coeur.

6. Dispositif selon la revendication 1, dans lequel ledit dispositif (30), une fois déployé, est adapté pour réduire un volume de ladite chambre.

7. Dispositif selon la revendication 1, dans lequel ledit dispositif (30), une fois déployé, est adapté pour cloisonner ladite chambre dudit coeur en une partie fonctionnelle et une partie non fonctionnelle.

8. Dispositif selon la revendication 1, dans lequel lesdites nervures (34) de longueur différente sont autodilatables lors du déploiement.

9. Dispositif selon la revendication 1, dans lequel lesdites nervures pouvant se dilater radialement (34) se dilatent jusqu'à un diamètre proximal d'environ 5 mm à environ 80 mm.

10. Dispositif selon la revendication 1, dans lequel lesdites nervures (34) de longueur différente dudit dispositif (30) fournissent une force vers l'extérieur contre une paroi de ladite chambre.

11. Dispositif selon la revendication 1, dans lequel ledit dispositif (30), une fois déployé, augmente le mouvement d'une paroi de ladite chambre.

12. Dispositif selon la revendication 1, dans lequel ledit dispositif (30) est adapté pour réduire la régurgitation mitrale aiguë.

13. Dispositif selon la revendication 1, comprenant en outre une tige (43) s'étendant distalement à partir desdites extrémités distales (36) desdites nervures (34) de longueur différente, dans lequel ladite tige est adaptée pour espacer ledit dispositif (30) d'une distance sélectionnée par rapport à une paroi de ladite chambre dudit coeur.

14. Dispositif selon la revendication 2, dans lequel ladite membrane (31) forme une surface de réception de pression (35).

15. Dispositif selon la revendication 4, dans lequel le ou chaque élément d'ancrage (48) perce un tissu à un angle de pas plus de 45° par rapport à un axe de ligne centrale (38) dudit dispositif (30).

16. Dispositif selon la revendication 7, dans lequel ladite partie non fonctionnelle comprend une rupture myocardique.

17. Dispositif selon la revendication 7, dans lequel ledit dispositif (30) est adapté pour cloisonner une rupture myocardique qui n'est pas située au niveau d'un apex d'un ventricule.

18. Dispositif selon la revendication 7, dans lequel ladite partie non fonctionnelle comprend un pseudo-anévrisme.

19. Dispositif selon la revendication 9, dans lequel ledit diamètre proximal dudit dispositif (30) est surdimensionné en comparaison à un diamètre de ladite chambre dudit coeur.

20. Dispositif selon la revendication 13, dans lequel ladite tige (43) comprend un composant de support non traumatique (44) raccordé au niveau de son extrémité distale.

21. Dispositif selon la revendication 14, dans lequel ladite surface de réception de pression (35) est concave au niveau de son extrémité distale.

22. Dispositif selon la revendication 16, dans lequel ladite rupture myocardique est une communication intraventriculaire.

23. Dispositif selon la revendication 16, dans lequel ladite rupture myocardique est une tamponnade péricardique cardiaque.

24. Dispositif selon la revendication 20, dans lequel ledit composant de support non traumatique (44) comprend une pluralité de pattes (45) adaptée pour répartir une force du dispositif (30) autour d'une région de ladite paroi de ladite chambre.
